# EUROPEAN PATENT APPLICATION

(11) **EP 1 571 213 A1**
(43) Date of publication of application: **07.09.2005**
(21) Application number: 04292299.7
(22) Date of filing: 24.09.2004
(51) Int. Cl.: C12N 15/31, C12N 1/20, C07K 14/195, A61K 38/16, A61K 39/02, C12R 1/01

(54) **Bifidobacterium longum AR81 (KCCM-10492) enabling inhibition of rotavirus and active protein separated therefrom**

(30) Priority: 26.09.2003 KR 2003067066
(71) Applicant: Bifido Co., Ltd., Gangwon-do (KR)
(72) Inventor: Ji, Geun-Eog, Chuncheon-si Gangwon-do (KR); Park, Myeong-Soo, Chuncheon-si Gangwon-do (KR); Kim, Dong-Hyun, Chuncheon-si Gangwon-do (KR); Bae, Eun-Ah, Chuncheon-si Gangwon-do (KR)
(74) Representative: Geismar, Thierry

(57) **Abstract**

The present invention relates to a novel strain enabling inhibition of rotavirus that is known as a principal cause of acute diarrhea and enteritis in children and in the young animals of cattle, horses, pigs and monkeys, etc, and an active protein also having an anti-rotavirus activity produced from the same.

The novel strain of the present invention was named as *Bifidobacterium longum* AR81 (KCCM-10492).

The novel strain of the present invention and the active protein separated from the same can be effectively used for the production of medicine for intestinal disorders and especially for the production of baby goods.

## Description

### BACKGROUND OF THE INVENTION

### (a) Field of the Invention

The present invention relates to a novel strain having an activity of inhibiting rotavirus and an active protein separated therefrom, more precisely, a novel strain *Bifidobacterium longum* AR81 (KCCM-10492) and an active protein also having an anti-rotavirus activity produced from the same.

### (b) Description of the Related Art

A particle of rotavirus shape was found by Ms. Bishop, *et al.* (Australia) in 1973 in biopsy sample of duodenum of a child hospitalized for the treatment of acute diarrhea.

Rotavirus belongs to Family Reoviridae and was given the name 'rota' because its double capsid looks like a wheel of the cart. Ever since it was first found, rotavirus has been confirmed by numbers of research teams in the entire world to be a major cause of acute diarrhea in babies.

Then, rotavirus was separated by Light and Hodes in 1943 in feces of a baby having gastroenteritis. And the morphological characteristics of the virus were disclosed under electron microscope in 1977. In the meantime, reovirus-like virus was isolated from mammals and birds, and the virus was later confirmed to be rotavirus. So, the virus, rotavirus, was finally grouped into Genus Rotavirus belonging to Family Reoviridae.

Rotavirus is divided into 6 different antigen groups (A-f). Rotaviruses of group A, B and C are found in both human and animals, and rotaviruses of group D, E and F are found in animals only. Groups divided by serological characteristics have a common or a cross antigen. Rotavirus of group A has been studied most since it was first reported, so that its characteristics have been investigated thoroughly. Rotavirus of group A is largely distributed among human-derived strains, and subgroups are determined by its inner capsid protein VP6 and serological types are determined by outside capsid protein VP7 and VP4. At least two subgroups and four serological types have been disclosed so far.

Rotavirus has an outside capsid and the core without envelope. The viral genome is composed of 11 individual segments of double helical RNA. Outside capsid of the virus is composed of two proteins 'VP4 and VP7'. VP4, a cell adherent protein and a hemagglutinin, stands out like a spike from the surface of the virus and takes 2.5% of the total weight of the virus. VP4 is split into VP5* (Mᵣ 60,000) and VP8* (Mᵣ 28,000) to penetrate into inner cells. VP7 is a glycoprotein having the weight of 37 kDa, taking 30% of the total weight of the virus, and constructs cellular shell of the smooth outside capsid.

Table 1 below presents general characters of rotavirus.

**<Table 1>**

| | |
|---|---|
| Virion | Icosahedral |
| | Diameter: 60-80 nm |
| | Double capsid shell |
| Composition | RNA (15%), protein (85%) |
| Genome | 11 segments composed of double helical RNA |
| | Total molecular weight: 12-15 million Dalton |
| Protein | 9 structural proteins |
| | Core includes a couple of enzymes |
| Envelope | No |
| Replication | Cytoplasmic replication |

Rotavirus is known as a principal cause of acute diarrhea and enteritis in children and in the young animals of cattle, horses, pigs and monkeys. An infection route of the virus is from feces through the mouth. Rotavirus causes an infectious acute diarrhea in babies under age 2, which is so called infantile gastroenteritis or acute gastroenteritis.

Diarrhea is a result of troubles in absorption of intestinal mucosal epithelial cells caused by the infection with rotavirus. Once villus cells of small intestine are infected with the virus, the virus proliferates in cytoplasm of those cells to cause troubles in transport system. Damaged cells are apart and spout out the virus in intestinal track, so that the virus can be found in feces. When rotavirus infiltrates, damaged cells of villi are replaced by immature crypt cells not having absorption capability, causing malfunction of absorption of sodium and glucose, resulted in diarrhea.

Rotavirus induced diarrhea in babies mostly comes out briefly right after the beginning of weaning, which is called "Weanling diarrhea" or "Colibacillosis". Such diarrhea, especially in an aerobic strain, is inevitably accompanied with the transition to non-hemolytic *E. coli* and hemolytic *E. coli* of *Enterococci.* Hemolytic *E. coli* generates enterotoxin and harbors pili which enables *E. coli* to adhere to an enterocyte. Such strains are named enteropathogenic *E. coli* or enterotoxigenic E. coli, and have various serotypes.

It was reported in 1973 that a very serious diarrhea was developed in a piglet bred without colostrum. After investigating the cause of the disease, it was diagnosed as colibacillosis. The serious diarrhea in a piglet that was bred without colostrum was caused by the infection with diarrheal fluid harboring retrovirus destroying cells by infiltrating in enterocytes. And numbers of retrovirus were found in diarrheal feces of a three to four week old piglet in weaning period. It was important to find out the cause of diarrhea in a piglet and rotavirus was believed to be a relevant principal cause of the disease. It was also confirmed that non-hemolytic *E. coli* was transformed into hemolytic *E. coli* during diarrhea. However, when hemolytic and enteropathogenic *E. coli* were orally administered without rotavirus to a piglet, diarrhea didn't come out. In conclusion, rotavirus damages intestinal track to builds up a proper environment for enteropathogenic *E. coli* to move into small intestines and form a colony.

Clinical symptoms of a retrovirus induced disease are vomiting, diarrhea, abdominal pain and fever, which are mostly related to intestinal tracks. In particular, dehydration and electrolyte imbalance are progressed fast in babies.

According to the statistical data of World Health Organization (WHO), 5 million children under age 5 on earth die of diarrhea every year, and 20% (one million) of them died of rotavirus induced diarrhea. According to a report that investigated more than 12 possible causes of diarrhea for 12 months with children in Seoul, rotavirus was detected in 47% of children suffering from diarrhea, which was the highest appearance exceeding statistics of WHO (20%). Rotavirus induced diarrhea prevails in winter and is the leading cause of infant mortality in the developing countries. The best precaution to prevent the disease is breast-feeding. Since mother's milk includes IgA acting against rotavirus, infantile diarrhea caused by rotavirus can be prevented by breast-feeding.

Table 2 below presents the statistics about enteropathogenics of Korean children with and without diarrhea.

**<Table 2>**

| Enteropathogenic | No. (%) of confirmed enteropathogenic^{a} | |
|---|---|---|
| | Diarrhea group^{b} | Control group^{c} |
| Rotavirus | 107(46.5) | N.D. |
| Enterotoxigenic *E.coli* (ETEC) | 52(22.5) | 14(13.5) |
| Other ST *Enterobacteriaceae* | 9 (4.8) | 1(1.0) |
| Enteroadherent *E.coli* (EAEC) | 34(14.7) | 8(7.7) |
| Enteropathogenic *E.coli* (EPEC) | 15(6.5) | 5(4.8) |
| *Shigella* spp. | 4(1.7) | 0 |
| *Salmonella* spp. | 1(0.4) | 0 |
| *Campylobacter jejuni* | 1(0.4) | 0 |
| Enteroinvasive *E.coli* (EIEC) | 1 (0.4) | 0 |

| | | |
|---|---|---|
| ^{a} : Numbers of pathogens separated from one patient were all recorded. | | |
| ^{b} : n = 231 | | |
| ^{c} : n = 104 | | |

56 patients (24.2%) with diarrhea were confirmed not to have a pathogen, and 52 people (52.9%) in a control group were confirmed not to have a pathogen.

Most patients are well recovered by the supply with water, but it is not easy for patients of developing countries to be treated simply with water. Although the frequency of development of bacterial diarrhea decreases by the supply with fresh water and sanitation, rotavirus induced diarrhea does not much decreased.

Infection with rotavirus causes the synthesis of IgA secreted in lumen. According to the experiments with sheep, calves and pigs, an antibody secreted in lumen is more important to prevent rotavirus infection than any other antibody existing in serum or lymph.

Thus, it is preferred to develop a live virus vaccine for oral administration, and thus, attempts have been made to develop a subunit vaccine or a microencapsulated killed vaccine.

Every effort has been made for the past 20 years to develop a novel vaccine and both conventional and molecular biological methods have been tried so far. Nevertheless, the development of a novel vaccine without much side effect was not successful. A vaccine was once developed, but it was not good enough for the use because of serious side effects.

### SUMMARY OF THE INVENTION

The present inventors paid attention to the point that rotavirus infection shows up largely in the weaning period, during which the number of *Bifidobacterium* residing in intestines decreases rapidly but the number of *E. coli* increases.

And so, it is an object of the present invention to provide a novel *Bifidobacterium* strain *'Bifidobacterium longum* AR81 (KCCM-10492)' having an activity of inhibiting rotavirus infection, in order to solve the above-mentioned problem.

It is another object of the present invention to provide an active protein having an anti-rotavirus activity produced from the strain of the invention.

### BRIEF DESCRIPTION OF THE DRAWINGS

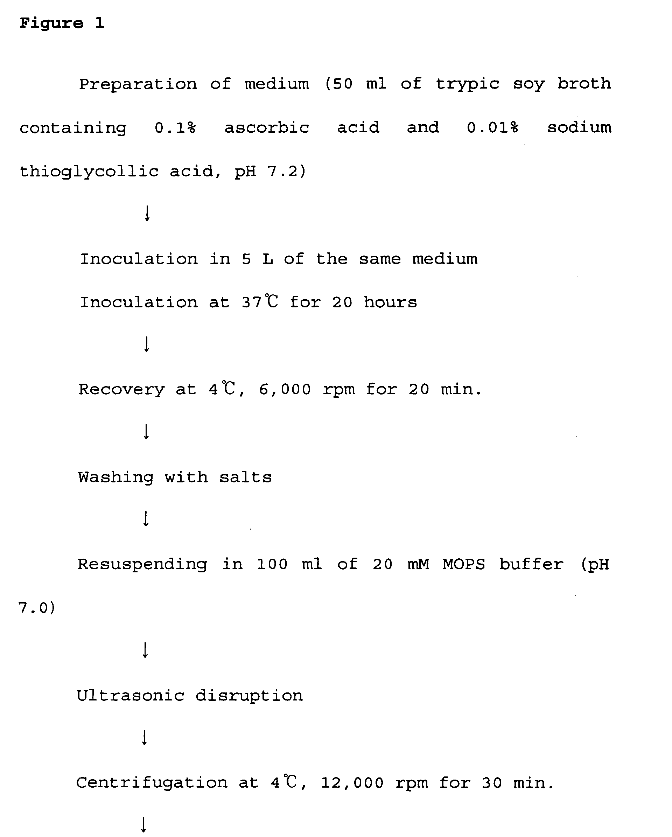

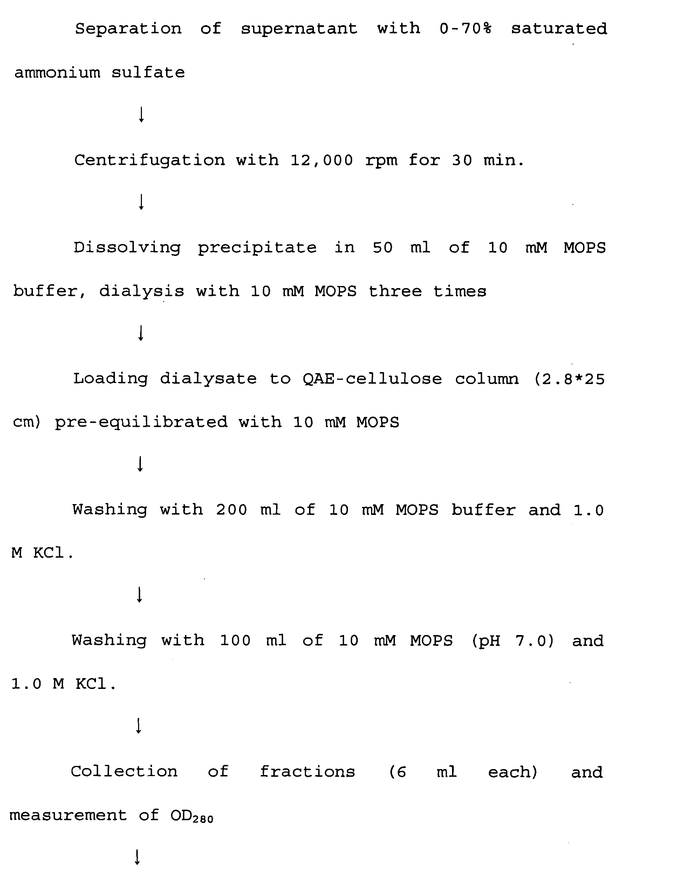

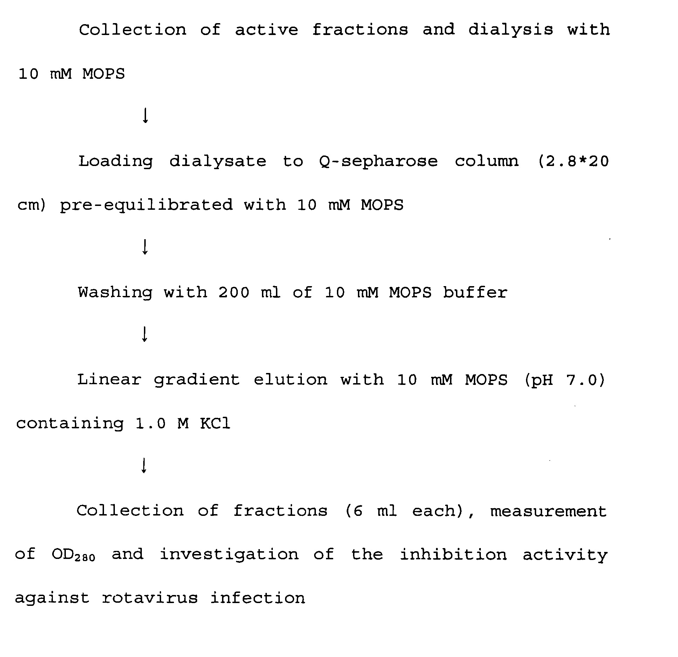

FIG. 1 is a schematic diagram showing the separation and the purification processes of the active protein of the present invention.

### DETAILED DESCRIPTION OF THE PREFERRED EMBODIMENTS

The present invention provides a novel *Bifidobacterium* strain '*Bifidobacterium longum* AR81 (KCCM-10492)' having an activity of inhibiting rotavirus and a protein acting against rotavirus which is produced from the same.

The novel *Bifidobacterium* strain inhibiting rotavirus of the present invention was named '*Bifidobacterium longum* AR81 (KCCM-10492) and was deposited at Korean Culture Center of Microorganisms (KCCM) on May 6, 2003 (Accession No: KCCM-10492).

The characteristics of *Bifidobacterium longum* AR81 (KCCM-10492) isolated by the present inventors are shown in below Table 3.

**<Table 3>**

| Characteristics of *Bifidobacterium longum* AR81 | |
|---|---|
| Morphology | Rods or Y-shaped |
| Gram staining | + |
| F6PPK | + |

Protein acting against rotavirus was separated from the strain and a nucleotide sequence of a gene coding the protein was represented by SEQ. ID. No 1. Amino acid sequence thereof was also represented by SEQ. ID. No 2.

The protein of the present invention was confirmed to act against rotavirus. A pharmaceutical composition containing the protein of the present invention can include pharmaceutically acceptable excipients, carriers, diluents, etc., and can be produced in the forms of tablets, pills, disperse preparation, granules, elixirs, suspensions, emulsions, solutions, syrups, etc. The composition can be administered either orally or parenterally and its dosage of a day is 1-20 mg/kg weight.

For the separation and purification of the protein, the novel strain of the invention '*Bifidobacterium longum* AR81 (KCCM-10492) was cultured in a trypic soy broth (pH 7.2) supplemented with 0.1% ascorbic acid and 0.01% sodium thioglycollic acid, followed by centrifugation, precipitation and dialysis.

Practical and presently preferred embodiments of the present invention are illustrative as shown in the following Examples.

However, it will be appreciated that those skilled in the art, on consideration of this disclosure, may make modifications and improvements within the spirit and scope of the present invention.

### <Example 1>

### Target cells and culture of the cells

The target cell line used in the present invention was Macaccus' Rhesus monkey kidney cell line (MA-104), which was provided from Toyama sanitary testing and research institute, Japan.

MA-104 cells were cultured in a 37°C 5% CO₂ incubator using DMEM supplemented with 10% FBS, 1% antibiotics-antifungal agent and 3.5 g/L of sodium bicarbonate.

### <Example 2>

### Distribution of rotavirus and preparation of the virus solution

SA11 rotavirus was provided from National Institute of Health (NIH), Korea and Wa rotavirus was provided from ATCC.

Each virus was distributed into T.C. flasks (25cm²) by the concentration of 1-2x10⁶ cells/flask, then cells were adhered for one hour under the condition of 37°C and 5% CO₂.

Supernatant was removed and the cells were washed with FBS-free DMEM medium. 20 µl of DMEM medium (infectious medium) containing 5 µg/ml of trypsin was added to 400 µl of Wa virus or SA11 virus solution, which was treated at 37°C for 30 minutes. 400 µl of pre-activated Wa virus solution was spread over the cell surface evenly, leading to infection at 37°C for one hour.

Upon completing the infection, supernatant was removed and cytopathic effect (cpe) was investigated. After confirmation, it was frozen.

Freezing-thawing was repeated three times to break the cell membrane completely. Centrifugation was performed at 4°C, 500 rpm for 20 minutes. Supernatant, a virus solution, was taken only and kept in a refrigerator.

### <Example 3>

### Lactobacillus and Bifidobacterium used in the present invention

*Lactobacillus acidophilus* KCTC 3150, *Bifidobacterium longum* KCTC 3215 and *Bifidobacterium infantis* KCTC 3226 used in the present invention were provided from Institute of Genetic Engineering, Korea, and the rest of the strains used in the invention were separated from bacterial flora residing in the human intestines.

### <Example 4>

### Determination of titer of rotavirus solution (end-point dilution method)

MA-104 cells cultured in the above Example 1 were treated with 0.25% trypsin-EDTA to isolate them. Then, a fresh medium was provided. Centrifugation was performed at 1200 rpm for 5 minutes to discard supernatant. Cell concentration was adjusted to 5x10⁵ cells/ml using infectious medium.

Rotavirus stock solution was serially diluted from 10⁻¹ to 10⁻⁸, and each diluted solution was distributed to 8 wells by 100 µl.

The cultured MA-104 cells were distributed thereto by the same amount, followed by further culture in a 37°C, 5% CO₂ incubator for 7 days.

Upon completing the culture, cpe of each well was investigated under inverted microscope to calculate TCID₅₀ (50% Tissue-culture infectious dose)/ml. Pfu (plaque forming unit)/ml was calculated by multiplying the value of TCID₅₀/ml by a coefficient 0.69.

### <Example 5>

### The effect of rotavirus infection on components of lactobacillus cytoplasm

Each strain used for the experiments was cultured in 5 ml of GAM broth for 18 hours. Centrifugation was performed at 3000 rpm for 20 minutes, resulting in the preparation of bacteria precipitate. The precipitate was suspended in 1 ml of PBS, followed by sonication. Centrifugation was then performed at 12000 rpm for 60 minutes to obtain supernatant, resulting in lactobacillus cytoplasm solution.

The prepared cytoplasm solution was filtered with 0.45 µm syringe filter for sterilization.

The cytoplasm solution of each strain was diluted to the concentrations of 0.01, 0.02 and 0.04 mg protein/ml, which was added by 50 µl to each well containing MA-104 cells and SA11 virus or Wa virus diluted solution, followed by further culture. Then, cpe was investigated and the results were shown in Table 4 and Table 5.

**<Table 4>**

| *Bifidobacterium* | Infection inhibition rate (%) | | |
|---|---|---|---|
| | Cytoplasmic component (mg/ml) | | |
| | 0.0005 | 0.002 | 0.010 |
| *B-47* | 16.7 | 33.3 | 66.6 |
| *B-81* | **0** | **33.3** | **100** |
| *B-180* | 0 | 16.7 | 83.3 |
| *KK-11* | 0 | 16.7 | 66.6 |
| *KK-12* | 0 | 16.7 | 83.3 |
| *B. longum*^{*a*} | 0 | 14.3 | 14.3 |
| *B. infantis*^{a} | 0 | 28.6 | 42.9 |
| *L. acidophilus*^{*a*} | 0 | 14.3 | 28.6 |
| *B-81 : Bifidobacterium longum* AR81 (KCCM-10492) | | | |

| | | | |
|---|---|---|---|
| a : KCTC | | | |

**<Table 5>**

| *Bifidobacterium* | Infection inhibition rate (%) | | |
|---|---|---|---|
| | Cytoplasmic component (mg/ml) | | |
| | 0.0005 | 0.002 | 0.010 |
| *B-47* | 16.7 | 16.7 | 66.6 |
| *B-81* | **0** | **33.3** | **100** |
| *B-179* | 0 | 33.3 | 83.3 |
| *KK-11* | 0 | 16.7 | 66.6 |
| KK-12 | 0 | 16.7 | 83.3 |
| *B. longum*^{*a*} | 0 | 14.3 | 14.3 |
| *B. infantis*^{*a*} | 0 | 28.6 | 42.9 |
| *L. acidophilus*^{*a*} | 0 | 14.3 | 42.9 |
| B-81 : *Bifidobacterium longum* AR81 (KCCM-10492) | | | |

| | | | |
|---|---|---|---|
| a : KCTC | | | |

Among lactic acid bacteria, cytoplasmic components of *Bifidobacterium animalis* KCTC 3126 and *Bifidobacterium infantis* KCTC 3226 were confirmed to have very good infection inhibition effect. Among isolated strains, 47, 81 (*Bifidobacterium longum* AR81 (KCCM-10492)) and 179 strains showed excellent inhibition effect, and in particular, strain 81, a novel strain of the present invention, showed the best inhibition effect.

Isolated strains, strain 47, 81 and 179, were confirmed to be Gram-positive. And their morphological characteristics are equal to those of *Bifidobacteria* (rods shaped, Y-shaped, etc.). After investigating the activity to *Bifidobacteria-specific* enzyme F6PPK (fructose 6 phosphate phosphoketolase), the above strains were all confirmed to be positive, indicating that they are all *Bifidobacteria.*

Among isolated strains, highly activated strain 47 and strain 81 were identified by 16S rDNA nucleotide sequence analysis. In order to identify a strain using 16S rDNA sequence, PCR primer had to be prepared first. The general 16S rDNA sequence of *Bifidobacterium* was obtained from Gene Bank, whose size was about 1.5 kb and preservative sequence was found in inside. Based on that sequence, 4 primers were constructed.

A template for PCR was prepared by using Genereleaser kit. Then, 0.5 kb long DNA fragment was prepared by using primers 616V and 610R in order to obtain a sequencing template. Then, 0.5 kb DNA was obtained by using 612F and 630RIII.

Electrophoresis was performed to recover DNA band using a gel extraction kit (Qiagen, U.S.A). TA cloning to pGEM T easy vector (Promega, USA) was performed for sequence confirmation.

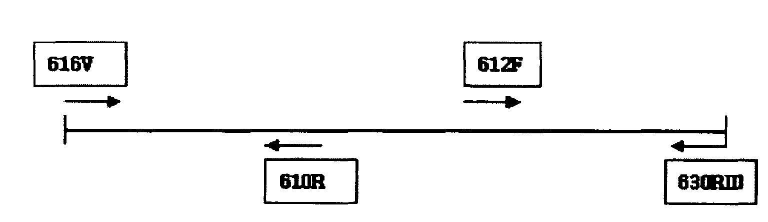

After comparing the sequences of strain 47 and strain 81 with others of Gene Bank, those sequences were confirmed to have 99% homology with that of *Bifidobacterium longum,* leading to the identification of them as *B. longum.* In particular, strain 81 was named '*Bifidobacterium longum* AR81 (KCM-10492)' and deposited at Korean Culture Center of Microorganisms (KCCM) on May 6, 2003.

Below are the information about primers to strain 47 and strain 81, a novel strain of the present invention.

Strain 47 (forward primer):
1. *Bifidobacterium longum* NCC2705 section 172 of 202 of the complete genome Length = 10301 Score = 1061 bits (535), Expect = 0.0 Identities = 553/559 (98%), Gaps = 1/559 (0%) Strand = Plus / Plus

Strain 47 (reverse primer) :
1. *Bifidobacterium longum* NCC2705 section 172 of 202 of the complete genome Length = 10301 Score = 940 bits (474), Expect = 0.0 Identities = 477/478 (99%) Strand = Plus / Plus

Strain 81 (forward primer):
1. *Bifidobacterium longum* NCC2705 section 172 of 202 of the complete genome Length = 10301 Score = 1061 bits (487), Expect = 0.0 Identities = 486/487 (99%), Gaps = 1/487 (0%) Strand = Plus / Plus

Strain 81 (reverse primer) :
1. *Bifidobacterium longum* NCC2705 section 172 of 202 of the complete genome Length = 10301 Score = 1061 bits (521), Expect = 0.0 Identities = 520/521 (99%), Gaps = 1/521(0%) Strand = Plus / Plus

### <Example 6>

### The effect of rotavirus infection on the components of lactobacillus cell wall

The strains used in the experiments were cultured in 5 ml of GAM broth for 18 hours. Then, centrifugation was performed at 3000 rpm for 20 minutes, resulting in the preparation of bacteria precipitate. The precipitate was suspended in 1 ml of PBS, followed by sonication. Centrifugation was performed again at 12000 rpm for 60 minutes to obtain precipitate, which was prepared as lactobacillus cell membrane and cell wall. The precipitate was suspended in 1 ml of PBS, to which the same amount of ether was added. The solution was left for 24 hours for sterilization.

The cytoplasmic solution of each strain was diluted to make final concentration to be 0.02, 0.04 and 0.08 mg protein/ml each, which was added by 50 µl to each well containing the diluted MA-104 cells and SA11 virus or Wa virus solution. After culturing, cpe was investigated, and the results are shown in Table 6 and Table 7.

**<Table 6>**

| *Bifidobacterium* | Infection inhibition rate (%) | | |
|---|---|---|---|
| | Cell wall (mg/ml) | | |
| | 0.0005 | 0.002 | 0.010 |
| *B-47* | 16.7 | 0 | 16.7 |
| ***B-81*** | **0** | **28.6** | **16.7** |
| *B-180* | 0 | 16.7 | 33.3 |
| *KK-11* | 16.7 | 0 | 16.7 |
| *KK-12* | 16.7 | 0 | 16.7 |
| *B. longum*^{*a*} | 0 | 14.3 | 0 |
| B. infantis^{a} | 0 | 14.3 | 0 |
| *L. acidophilus*^{*a*} | 0 | 0 | 0 |
| *B-81 . Bifidobacterium longum* AR81 (KCCM-10492) | | | |

| | | | |
|---|---|---|---|
| a : KCTC | | | |

**<Table 7>**

| *Bifidobacterium* | Infection inhibition rate (%) | | |
|---|---|---|---|
| | Cell wall (mg/ml) | | |
| | 0.0005 | 0.002 | 0.010 |
| *B-47* | 0 | 0 | 16.7 |
| ***B-81*** | **0** | **28.6** | **16.7** |
| *B-179* | 0 | 16.7 | 33.3 |
| *KK-11* | 16.7 | 16.7 | 16.7 |
| *KK-12* | 16.7 | 16.7 | 16.7 |
| *B. longum*^{*a*} | 0 | 14.3 | 14.3 |
| *B. infantis*^{*a*} | 0 | 14.3 | 14.3 |
| *L. acidophilus*^{*a*} | 14.3 | 14.3 | 14.3 |
| *B-81 : Bifidobacterium longum* AR81 (KCCM-10492) | | | |

| | | | |
|---|---|---|---|
| a : KCTC | | | |

As shown in Table 6 and Table 7, a cytoplasmic component of lactobacillus is better at inhibiting virus infection than a cell wall component.

### <Example 7>

### Characteristics and determination of Wa virus infection inhibitor included in lactobacillus

Lactobacillus was cultured in GAM broth. Cells were collected by centrifugation, which were suspended in physiological saline, followed by sonication. Centrifugation was performed again to obtain supernatant. The supernatant was autoclaved, filtered, heat-treated (100°C, 15 minutes), incinerated or precipitated with acetone.

The precipitate was diluted to make the final concentrations (V/V %) of 5, 10 and 20%, which were added by 50 µl to each well containing MA-104 cells and diluted rotavirus solution. Then cpe was investigated. The results are shown in Table 8.

**<Table 8>**

| *Lacto- bacillus* | Treatment | Inhibition rate (%) |
|---|---|---|
| B-47 | Autoclaving | 0 |
| | Heating | 0 |
| | Filtration | 66.6 |
| | Incineration | 0 |
| | Acetone precipitation | 50 |
| B-81 | Autoclaving | 16.7 |
| | Heating | 0 |
| | Filtration | 50 |
| | Incineration | 0 |
| | Acetone precipitation | 33.3 |
| KK-11 | Autoclaving | 0 |
| | Heating | 0 |
| | Filtration | 50 |
| | Incineration | 0 |
| | Acetone precipitation | 33.3 |
| ^{a} : Final concentration (%) | | |
| ^{b} : Not detected | | |

While virus infection inhibiting effect of lactobacillus decreased or disappeared by autoclaving, heating, incineration, etc., the effect slightly decreased by acetone precipitation and was sustained by filtering for sterilization. Thus, it was judged that a protein was the substance inhibiting Wa virus infection.

### <Example 8>

### Cloning of a gene of a protein having an anti-rotavirus activity

For the cloning of a gene of a protein having an anti-rotavirus activity, a protein was separated and purified from selected strains by the processes described in FIG. 1.

### <Example 9>

### Identification of an amino acid sequence of an active protein having an anti-rotavirus activity

Internal sequence assay was performed to identify an amino acid sequence of the protein, separated/purified in the above Example 8, having an anti-rotavirus activity.

The identification of an amino acid sequence was entrusted to Korea Basic Science Institute (KBSI), and as a result, an amino acid sequence composed of 10 amino acids was identified.

### N'-HLDLAVENT-C'

After data base (GenBank) search, a corresponding sequence to the identified amino acid sequence was found in dipeptidaseD (GenBank Accession number NC_004307), one of bifidus originated genes.

Based on that information, PCR was performed by using the below primers to amplify the region including a whole gene coding the above amino acid sequence.

pepD-F: 5'-AAAACCGATTGGCAACTCGGG-3
pepD-R: 5'-GCCACTTCTGGGCGCCGGC-3'

Then, the PCR product was cloned into pGEM-Teasy vector to identify nucleotide sequence.

The nucleotide sequence is represented by SEQ. ID. No 1, and the protein thereof is represented by SEQ. ID. No 2.

### <Example 10>

### Expression of a protein having an anti-rotavirus activity and investigation of titer thereof

In order to investigate an anti-rotavirus activity of the protein of Example 7, the expression of the protein was investigated by using *E. coli* expression vector.

PCR was performed by using the below primers, and the product was linked to pBAD-TOPO.

pepTOPO-F: 5'-ATGGCCTGCACCACGATTCTGGTAGG-3'

pepTOPO-R: 5'-AAAGTCGGACATGTGGAAGCCGTTC-3'

*E. coli* was transfected with the vector. Then, the expression of the inserted gene was induced by using arabinose. Cells were recovered. Coenzyme solution was prepared by ultrasonic disruption. Then, an anti-rotavirus activity was investigated.

As shown in Table 9, an anti-rotavirus activity of the protein was higher than that of a control.

**<Table 9>**

| Agent | Infection inhibition rate (%) | |
|---|---|---|
| | 10X dilution | 100X dilution |
| Control | 25 | 0 |
| Transfected sample | 62.5 | 10 |

### <Example 11>

### Development of powdered medicine for intestinal disorders

Medicine for intestinal disorders for babies has been developed by using the novel strain of the present invention, which has an activity of inhibiting rotavirus.

First, the strain was inoculated to medium for bifidus production, followed by further culture (10 ml --> 500 ml --> 5 L --> 50 L --> 500 L --> 5 KL). Centrifugation was performed to recover cells. The recovered cells were mixed with cryoprotectants, followed by quick-freeze and complete drying with a lyophilizer. Cells of lactobacillus for intestinal regulation were also recovered by the same processes as described above and then used for the production of medicine for intestinal disorders. In order to give intestinal regulating effect and other functional effects, the strain of the present invention can be mixed with galactooligosaccharides and other components according to the below composition.

**<Table 10>**

| Component | Content |
|---|---|
| Bifidus raw powder | 8% |
| Lactobacillus raw powder | 1% |
| Cyclodextrin | 28% |
| Galactooligosaccharide | 12% |
| Alpha Corn | 30.5% |
| Aloe Vera | 0.5% |
| Glycerine Fatty Acid Ester | 16% |
| Xilytole | 1% |
| Calcium Lactate | 3% |
| Lactoferrin concentrate | 0.05% |
| Vitamin B2 | 0.04% |
| Vitamin B12 | 0.00004% |
| Vitamin D3 | 0.00006% |

As explained hereinbefore, the novel *Bifidobacterium* strain of the present invention '*Bifidobacterium longum* AR81 (KCCM-10492)' has an activity of inhibiting rotavirus that has been known as a principal cause of diarrhea in infants and in the young animals.

Particularly, the strain above and a protein having an anti-rotavirus activity separated from the same are useful for the production of medicine for intestinal disorders.

Moreover, the strain of the present invention and the protein separated therefrom can be used for the production of functional fermented milk for infants.

## Claims

1. A novel strain *Bifidobacterium longum* AR81 (KCCM-10492) having an activity of inhibiting rotavirus.

2. A gene encoding a protein having an anti-rotavirus activity separated from the *Bifidobacterium longum* AR81 (KCCM-10492) of claim 1 and having a nucleotide sequence represented by SEQ. ID. No 1.

3. A protein having an anti-rotavirus activity separated from the *Bifidobacterium longum* AR81 (KCCM-10492) of claim 1 and having an amino acid sequence represented by SEQ. ID. No 2.

4. A medicine for intestinal disorders containing the novel strain of claim 1 or the protein of claim 3 as an effective ingredient.

5. The medicine for intestinal disorders as set forth in claim 4, wherein the form of the medicine is selected from a group consisting of liquid and powder.

6. The medicine for intestinal disorders as set forth in claim 5, wherein the medicine is for infants.


SEQUENCING LIST

<110>   Bifido Co., Ltd.

<120>   Bifidobacterium longum AR81(KCCM-10492) enabling
inhibition of
        rotavirus and active protein separated therefrom

<160>   2

<170>   KopatentIn 1.71

<210>   1
<211>   3590
<212>   DNA
<213>   Bifidobacterium longum AR81

<220>
<221>   CDS
<222>   (971)..(2569)

<400>   1
   aaaaccgatt ggcaactcgg gaatatacag gcggcggcac cgatgcctct cacatcaccg
   60
   ggaaacgatg acggcaccgg ggaaaaccat cagcccacac tgcggaaaaa cactgccgaa
   120
   cctgtcgaac aaacattgaa gcaaaacact cataacccga cgcaactagc ccccctctga
   180
   tgagtggggg ctgtcgagcg tagcgagact gggggagaga ccccgtaacg cacagccccc
   240
   agtacgtcac acatatgagc gatactggga cgtatgaaga tttccgccga tttcaccgtc
   300
   atcccggacg atttcgccaa ggccgcggcc cctgaatatt tcaatggtgg cgtgcctgtg
   360
   gtctccttcc cgttctacat cgatgatgtg gatcccgagg cccgctacct gcactgggag
   420
   ttcgtggacc cggattcgat tccggtctgc ggcttcgagt ggattcactg gtccgtggcc
   480
   aatctgccga ttgatgcgct gatgtacgac ttcaatgatt cgcatgcgtt ggccatcccg
   540
   ccggacttct cccgtcagct gccggccatg attcccgaga ccgtgcaggg gcgcaactcc
   600
   tcggcctcca agttcttggg ccgcagcact gacccggcgg tgatcatgcg gtacaacggt
   660
   ccgcagccgc cggacaagga tcatgagtac tacctgcagg tgtgggccac caagaagccg
   720
   ttgcccggct tgaatcaggg cttctggctc aacgagctgt tgcacgcgct gcgcaacagc
   780
   ggtgaagtgc ccgacaccga cggcatcttc ctgaccggca aggcgtgagc cacatgagcc
   840
   cccgcaggcg ggggctgtca gcatcactga ctgggggtgg gtcagtacaa aaccaccttc
   900
   agtccgactt cgtcgtccag ctcccgccag tgggagcgtg actacaacaa agaaaggaca
   960
   ccatcacact atg gcc tgc acc acg att ctg gta ggc aaa gac gcg agc tat
   1012
   Met Ala Cys Thr Thr Ile Leu Val Gly Lys Asp Ala Ser Tyr
   1 5 10
   gac ggc tcg acc atc atc gcc cgc aac gag gat tcc gcg aac ggc gag
   1060
   Asp Gly Ser Thr Ile Ile Ala Arg Asn Glu Asp Ser Ala Asn Gly Glu
   15 20 25 30
   ttc aac ccg aag cgc ttc atc gtc gtg aag ccg gag gaa cag ccg cgc
   1108
   Phe Asn Pro Lys Arg Phe Ile Val Val Lys Pro Glu Glu Gln Pro Arg
   35 40 45
   gaa tac aag tcg gtg atc tcc cac cta acc atc acc ctg ccg gac gac
   1156
   Glu Tyr Lys Ser Val Ile Ser His Leu Thr Ile Thr Leu Pro Asp Asp
   50 55 60
   ccg ctg cag tac acc gcc gtg ccg aac gcg gac ctc aag gaa ggt att
   1204
   Pro Leu Gln Tyr Thr Ala Val Pro Asn Ala Asp Leu Lys Glu Gly Ile
   65 70 75
   tgg ggc gag gcc ggc gtc aac gag gcg aac gtg gcg atg agc gcc acc
   1252
   Trp Gly Glu Ala Gly Val Asn Glu Ala Asn Val Ala Met Ser Ala Thr
   80 85 90
   gaa acc ctg acc acc aac gag cgc gtg ctc ggc gcc gac ccg ttc gtg
   1300
   Glu Thr Leu Thr Thr Asn Glu Arg Val Leu Gly Ala Asp Pro Phe Val
   95 100 105 110
   gaa tac acg ccg gcc aag ggc gat gag ccc gag gtg ccc ggc ggc atc
   1348
   Glu Tyr Thr Pro Ala Lys Gly Asp Glu Pro Glu Val Pro Gly Gly Ile
   115 120 125
   ggc gaa gag gac ttc ctg acc atc gtg ctg ccg tat gtc aag acc gca
   1396
   Gly Glu Glu Asp Phe Leu Thr Ile Val Leu Pro Tyr Val Lys Thr Ala
   130 135 140
   cgc gag ggc gtc cag cgc ctc ggc gcg ctg ctt gag gaa ttc ggc acc
   1444
   Arg Glu Gly Val Gln Arg Leu Gly Ala Leu Leu Glu Glu Phe Gly Thr
   145 150 155
   tac gag atg aac ggc gtc gcc ttc tcc gac tcc aac gaa atc tgg tgg
   1492
   Tyr Glu Met Asn Gly Val Ala Phe Ser Asp Ser Asn Glu Ile Trp Trp
   160 165 170
   ctg gaa act gtg ggc ggc cat cac tgg att gcc aag cgc gtg ccg gac
   1540
   Leu Glu Thr Val Gly Gly His His Trp Ile Ala Lys Arg Val Pro Asp
   175                 180                 185                 190
   gag gcc tac gtg acc atg ccg aac cag ctc ggt atc gac gaa ttc gac
   1588
   Glu Ala Tyr Val Thr Met Pro Asn Gln Leu Gly Ile Asp Glu Phe Asp
                   195                 200                 205
   ctt gag gac gct ctg ggt gac cag gaa gcg cac atg tgt tcc gaa gac
   1636
   Leu Glu Asp Ala Leu Gly Asp Gln Glu Ala His Met Cys Ser Glu Asp
               210                 215                 220
   ctt gcc gaa ttc atc gaa acc aac cat ttg gac ctc gcg gtg gag aac
   1684
   Leu Ala Glu Phe Ile Glu Thr Asn His Leu Asp Leu Ala Val Glu Asn
           225                 230                 235
   act acc ccg ttc aac ccg cgc gat gcc ttc ggc tcc cac tct gac tcc
   1732
   Thr Thr Pro Phe Asn Pro Arg Asp Ala Phe Gly Ser His Ser Asp Ser
       240                 245                 250
   gac cac gtg tac aac acc ccg cgc gcc tgg tac atg cag cgc ttc ctc
   1780
   Asp His Val Tyr Asn Thr Pro Arg Ala Trp Tyr Met Gln Arg Phe Leu
   255 260 265 270
   aac ccg tac gac gag gtg tgg gac ggc ccg gac gcc gac cac aag ccc
   1828
   Asn Pro Tyr Asp Glu Val Trp Asp Gly Pro Asp Ala Asp His Lys Pro
   275 280 285
   acc tcc gac gac atc ccg tgg gcc cgt cag cct gag cgc aag gtg acc
   1876
   Thr Ser Asp Asp Ile Pro Trp Ala Arg Gln Pro Glu Arg Lys Val Thr
   290 295 300
   atc gag gat atc aag tac gtg ctg agc tcc cac tac cag ggc acc ccg
   1924
   Ile Glu Asp Ile Lys Tyr Val Leu Ser Ser His Tyr Gln Gly Thr Pro
   305 310 315
   ttc gat ccg tac ggc cag ctg ggc gat gag cgc acc cgc cac atg tac
   1972
   Phe Asp Pro Tyr Gly Gln Leu Gly Asp Glu Arg Thr Arg His Met Tyr
   320 325 330
   cgc acg atc ggc atc aac cgc cag agc cag ctc gcg gtg atg cag atc
   2020
   Arg Thr Ile Gly Ile Asn Arg Gln Ser Gln Leu Ala Val Met Gln Ile
   335                 340                 345                 350
   cgc ccg tac cgc ccg cag gcc tcc cgc gcc atc cag tgg atg gca tac
   2068
   Arg Pro Tyr Arg Pro Gln Ala Ser Arg Ala Ile Gln Trp Met Ala Tyr
                   355                 360                 365
   ggc tcc aac ccg ttc aac acg ctg gta ccg ttc ttc ccg aac gtg gac
   2116
   Gly Ser Asn Pro Phe Asn Thr Leu Val Pro Phe Phe Pro Asn Val Asp
               370                 375                 380
   acc acg ccc gcc tac ctt gag gac acc acc acc cgc gtg acg tcc gag
   2164
   Thr Thr Pro Ala Tyr Leu Glu Asp Thr Thr Thr Arg Val Thr Ser Glu
           385                 390                 395
   aac ttc tac tgg gcc aac cgc atc atc gcc gcc ctg tgc gat ggc gcc
   2212
   Asn Phe Tyr Trp Ala Asn Arg Ile Ile Ala Ala Leu Cys Asp Gly Ala
       400                 405                 410
   ttc cgc tcc acg tcc aac gcc gtt gag cgc tac cag gag aag acc ggt
   2260
   Phe Arg Ser Thr Ser Asn Ala Val Glu Arg Tyr Gln Glu Lys Thr Gly
   415 420 425 430
   gcg atg ggc cac cgc ttg gtc gcc gcc acc gac gag cag atc gcc cgt
   2308
   Ala Met Gly His Arg Leu Val Ala Ala Thr Asp Glu Gln Ile Ala Arg
   435 440 445
   ctg ggc ttg acg gcc gcc gag gaa gcc gcg cag tcc gcc gct gaa gag
   2356
   Leu Gly Leu Thr Ala Ala Glu Glu Ala Ala Gln Ser Ala Ala Glu Glu
   450 455 460
   gaa ttt gag gcc gac aac gtg gat ggc gat gtg cag ccg atg acc ccg
   2404
   Glu Phe Glu Ala Asp Asn Val Asp Gly Asp Val Gln Pro Met Thr Pro
   465 470 475
   gac gag acc atc gcc gcg ctg cgt aac ccc gag gtg cgc gag att ctc
   2452
   Asp Glu Thr Ile Ala Ala Leu Arg Asn Pro Glu Val Arg Glu Ile Leu
   480 485 490
   gcc gcc gcc aac cag acc atg gcc gac cag ctc aag gag gaa acc gaa
   2500
   Ala Ala Ala Asn Gln Thr Met Ala Asp Gln Leu Lys Glu Glu Thr Glu
   495                 500                 505                 510
   aag ctg ctt gac tcc gtg ctg tac act cgc agc atg gaa atg aag aac
   2548
   Lys Leu Leu Asp Ser Val Leu Tyr Thr Arg Ser Met Glu Met Lys Asn
                   515                 520                 525
   ggc ttc cac atg tcc gac ttt   t agtccgtatt gagctcccct ccctgagggg
   2600
   Gly Phe His Met Ser Asp Phe
               530
   agctgaccgc gaagcggact gagggggcta agccgttaag aaaacagccc agtgggctgt
   2660
   ttttaggcga agaggcggcg gcagccgccc caccaccccg ccccaccatc atcaacacag
   2720
   aaaggcttta tatgaccacc atcgaagact tcaccagcca gtacggtctg gtccagaaga
   2780
   tcgacgcatt cggctacatg aagtacctgg ccgagaaccc tgacgccccg cgcaagcacg
   2840
   gcaaggtggt gctcgtcacc gccgatacgc cgctcaaggc atcgcgcgga gaaggcaaga
   2900
   ccaccacgac catcgccctg atcgacgccc tgaacaaacg cggcattgat gcgaccgccg
   2960
   tgctgcgtca gccgagcatg ggcatcaccg cagccggttc caagggcggt gcctccggcg
   3020
   gcggcaaggc ttccctgacc caccccgagc tcatcgactg gggtctgtgc ggcgaaatgg
   3080
   gcgccatcga ggcggcccag aacctgctcg tctccttcgc cgagaaggcc atcgatgacg
   3140
   gcaagctcga cacgattctc gtgccgcgtg tctccgaggt gccgagccgc tccctgcgct
   3200
   ccaccgccgt ggactacggc aagggcaacg tgccggagaa ggtcgtgctc acgccgacct
   3260
   gcgagctcat gcagattgtg gtgctctccc gctcgatgga cgagatttcc gagcgcgtgt
   3320
   ccaagatgat cgccggcacc aaggacggca aggccgtgac cttcggcgaa ttcgtggacc
   3380
   tgtggcgtat cactggcatt ctggccgacg cggtcaagcc ggccaagacc gagaccgtaa
   3440
   acggctcccc ggtctacgtg cacggtggcc cgttcgccaa cgtgtccatc ggcattccga
   3500
   cgctggtctc cgtggaaatg gcctgcgccc tgcatgacgt ggtcatcgtc gaggccggtt
   3560
   acggcaccga cgccggcgcc cagaagtggc
   3590

<210> 2

<211> 533

<212> PRT

<213> Bifidobacterium longum AR81

<400> 2

Met Ala Cys Thr Thr Ile Leu Val Gly Lys Asp Ala Ser Tyr Asp Gly

1               5                   10                  15

Ser Thr Ile Ile Ala Arg Asn Glu Asp Ser Ala Asn Gly Glu Phe Asn
            20                  25                  30
Pro Lys Arg Phe Ile Val Val Lys Pro Glu Glu Gln Pro Arg Glu Tyr
        35                  40                  45
Lys Ser Val Ile Ser His Leu Thr Ile Thr Leu Pro Asp Asp Pro Leu
    50                  55                  60
Gln Tyr Thr Ala Val Pro Asn Ala Asp Leu Lys Glu Gly Ile Trp Gly
65                  70                  75                  80
Glu Ala Gly Val Asn Glu Ala Asn Val Ala Met Ser Ala Thr Glu Thr
                85                  90                  95
Leu Thr Thr Asn Glu Arg Val Leu Gly Ala Asp Pro Phe Val Glu Tyr
            100                 105                 110
Thr Pro Ala Lys Gly Asp Glu Pro Glu Val Pro Gly Gly Ile Gly Glu
        115                 120                 125
Glu Asp Phe Leu Thr Ile Val Leu Pro Tyr Val Lys Thr Ala Arg Glu
    130                 135                 140
Gly Val Gln Arg Leu Gly Ala Leu Leu Glu Glu Phe Gly Thr Tyr Glu

145                 150                 155                 160

Met Asn Gly Val Ala Phe Ser Asp Ser Asn Glu Ile Trp Trp Leu Glu

                165                 170                 175

Thr Val Gly Gly His His Trp Ile Ala Lys Arg Val Pro Asp Glu Ala

            180                 185                 190

Tyr Val Thr Met Pro Asn Gln Leu Gly Ile Asp Glu Phe Asp Leu Glu

        195                 200                 205

Asp Ala Leu Gly Asp Gln Glu Ala His Met Cys Ser Glu Asp Leu Ala

    210                 215                 220

Glu Phe Ile Glu Thr Asn His Leu Asp Leu Ala Val Glu Asn Thr Thr

225                 230                 235                 240

Pro Phe Asn Pro Arg Asp Ala Phe Gly Ser His Ser Asp Ser Asp His

                245                 250                 255

Val Tyr Asn Thr Pro Arg Ala Trp Tyr Met Gln Arg Phe Leu Asn Pro

            260                 265                 270

Tyr Asp Glu Val Trp Asp Gly Pro Asp Ala Asp His Lys Pro Thr Ser

        275                 280                 285

Asp Asp Ile Pro Trp Ala Arg Gln Pro Glu Arg Lys Val Thr Ile Glu
    290                 295                 300
Asp Ile Lys Tyr Val Leu Ser Ser His Tyr Gln Gly Thr Pro Phe Asp
305                 310                 315                 320
Pro Tyr Gly Gln Leu Gly Asp Glu Arg Thr Arg His Met Tyr Arg Thr
                325                 330                 335
Ile Gly Ile Asn Arg Gln Ser Gln Leu Ala Val Met Gln Ile Arg Pro
            340                 345                 350
Tyr Arg Pro Gln Ala Ser Arg Ala Ile Gln Trp Met Ala Tyr Gly Ser
        355                 360                 365
Asn Pro Phe Asn Thr Leu Val Pro Phe Phe Pro Asn Val Asp Thr Thr
    370                 375                 380
Pro Ala Tyr Leu Glu Asp Thr Thr Thr Arg Val Thr Ser Glu Asn Phe
385                 390                 395                 400
Tyr Trp Ala Asn Arg Ile Ile Ala Ala Leu Cys Asp Gly Ala Phe Arg
                405                 410                 415

Ser Thr Ser Asn Ala Val Glu Arg Tyr Gln Glu Lys Thr Gly Ala Met
            420                 425                 430

Gly His Arg Leu Val Ala Ala Thr Asp Glu Gln Ile Ala Arg Leu Gly
        435                 440                 445

Leu Thr Ala Ala Glu Glu Ala Ala Gln Ser Ala Ala Glu Glu Glu Phe
    450                 455                 460

Glu Ala Asp Asn Val Asp Gly Asp Val Gln Pro Met Thr Pro Asp Glu
465                 470                 475                 480

Thr Ile Ala Ala Leu Arg Asn Pro Glu Val Arg Glu Ile Leu Ala Ala
                485                 490                 495

Ala Asn Gln Thr Met Ala Asp Gln Leu Lys Glu Glu Thr Glu Lys Leu
            500                 505                 510

Leu Asp Ser Val Leu Tyr Thr Arg Ser Met Glu Met Lys Asn Gly Phe
        515                 520                 525

His Met Ser Asp Phe
    530